# FASCICULE DE BREVET EUROPEEN

(11) **EP 1 721 582 B1**
(45) Date de publication et mention de la délivrance du brevet: **23.09.2009**
(21) Numéro de dépôt: 06290701.9
(22) Date de dépôt: 03.05.2006
(51) Int. Cl.: A61B 19/02, A61M 35/00, B65D 77/04, B65D 77/20

(54) **Dispositif pour la préparation d'un site opératoire**
Vorrichtung zum Vorbereiten der Hautoberfläche bei chirurgischen Eingriffen
Device for the preparation of a surgical site

(30) Priorité: 13.05.2005 FR 0504821
(43) Date de publication de la demande: 15.11.2006
(73) Titulaire: VYGON, F-95440 Ecouen (FR)
(72) Inventeur: Carrez, Jean-Luc, 95440 Ecouen (FR); Algrain, Isabelle, 95380 Puiseux en France (FR); Lestoquoy, Patrick, 59551 Attiches (FR); Hocq, Xavier, 59530 Villers Pol (FR)
(74) Mandataire: Texier, Christian

(56) Documents cités:
- WO-A-02/083021
- US-A- 3 730 338
- US-A- 3 976 195
- US-A- 4 844 259
- US-A- 4 989 733
- US-A- 5 178 282
- US-A- 5 779 053

## Description

L'invention concerne la préparation d'un site cutané au bloc opératoire.

Cette préparation implique le concours d'un intervenant non stérile qui assure des opérations préliminaires de détersion et de badigeonnage, et d'un intervenant stérile qui assure une opération finale de badigeonnage, chaque opération nécessitant l'emploi d'un matériel et d'un liquide spécifique, ces matériels et ces liquides pouvant d'ailleurs varier selon les établissements hospitaliers.

On a proposé de nombreux dispositifs pour faciliter la préparation ou la réalisation d'une opération chirurgicale en regroupant certains des matériels à utiliser dans des blisters, (entre autres brevets US 3 730 338, US 4 844 259, US 4 989 733, WO 02/083021, US 3 976 195 et US 5 779 053) mais aucun des ces dispositifs n'a été conçu pour le protocole d'intervention visé par la présente invention qui implique le concours d'un intervenant non stérile et d'un intervenant stérile pour la préparation d'un site cutané au bloc opératoire.

Le préambule de la première revendication est basé sur le document US 3 730 338.

Pour faciliter la mise à disposition de ces matériels au bloc opératoire, avec un gain de temps et une meilleure sécurité, la présente invention préconise un dispositif caractérisé en ce qu'il comprend un matériel de détersion pour le lavage du site, un matériel pour une opération préliminaire de badigeonnage, et un matériel pour une opération finale de badigeonnage, en ce que ces matériels sont placés dans des conteneurs individuels qui sont réunis dans un conteneur d'ensemble dans des conditions stériles, et en ce que le conteneur du matériel destiné à l'opération finale de badigeonnage est scellé et isolé dans une enveloppe individuelle scellée, en sorte que les opérations préliminaires de détersion et de badigeonnage du site puissent être pratiquées par un intervenant non stérile et en ce que l'opération finale de badigeonnage du site puisse être pratiquée par un intervenant stérile.

Dans des réalisations préférées, on choisit ledit conteneur d'ensemble dans le groupe constitué par les enveloppes souples et les sachets ou, de préférence un bac rigide ou semi-rigide fermé sur le dessus par un film pelable ou déchirable.

Dans des réalisations préférées :
- le matériel de détersion comprend une cupule pour recevoir un produit de lavage, un gant de toilette pour nettoyer la peau du patient avec ce liquide sur et largement autour du site opératoire, une brosse pour nettoyer pour les parties plus difficiles à nettoyer et des serviettes pour sécher la peau ;
- le conteneur du matériel de détersion est un bac conformé pour présenter une première cavité apte à constituer la cupule et à recevoir la brosse pour nettoyer et une deuxième cavité apte à loger les serviettes et le gant ;
- chacun des matériels de badigeonnage comprend une cupule pour recevoir un produit désinfectant et une brosse pour badigeonner à tremper dans ce produit, cette brosse comprenant un patin et un manche ;
- le conteneur de chaque matériel de badigeonnage est un bac conformé pour présenter une cavité apte à constituer la cupule et à loger le patin de la brosse pour badigeonner, et une autre cavité apte à loger le manche de la brosse pour badigeonner ;
- chaque bac contenant un matériel de badigeonnage est fermé par un film pelable ou déchirable transparent ;
- l'un des bacs qui contient un matériel de badigeonnage est lui-même contenu dans un bac fermé par un film pelable ou déchirable ;
- le matériel de détersion et l'un des matériels de badigeonnage sont contenus dans des bacs individuels qui ne sont pas fermés mais sont enveloppés ensemble par un champ opératoire en non-tissé ;
- le bac contenant le matériel de détersion et les bacs contenant chacun un matériel de badigeonnage sont fermés chacun par un film pelable ou déchirable ;
- le conteneur d'ensemble est un bac fermé par un film pelable.
- sont empilés dans le conteneur d'ensemble du bas vers le haut le conteneur du matériel pour l'opération finale de badigeonnage, le conteneur du matériel pour l'opération préliminaire de badigeonnage et le conteneur (50) du matériel de détersion.

On décrira ci-après diverses réalisations de ce dispositif en référence aux figures du dessin joint sur lequel :
- la figure 1 est un schéma d'un premier exemple de réalisation d'un dispositif conforme à l'invention ;
- la figure 2 est un schéma d'une variante de réalisation du dispositif ;
- la figure 3 est un schéma en perspective éclatée d'un exemple de réalisation du matériel de détersion ;
- la figure 4 est un schéma en perspective du matériel de détersion de la figure 3 dans son conteneur individuel fermé par un film transparent ;
- la figure 5 est un schéma d'un ensemble de matériel de badigeonnage, vu en perspective éclatée, et

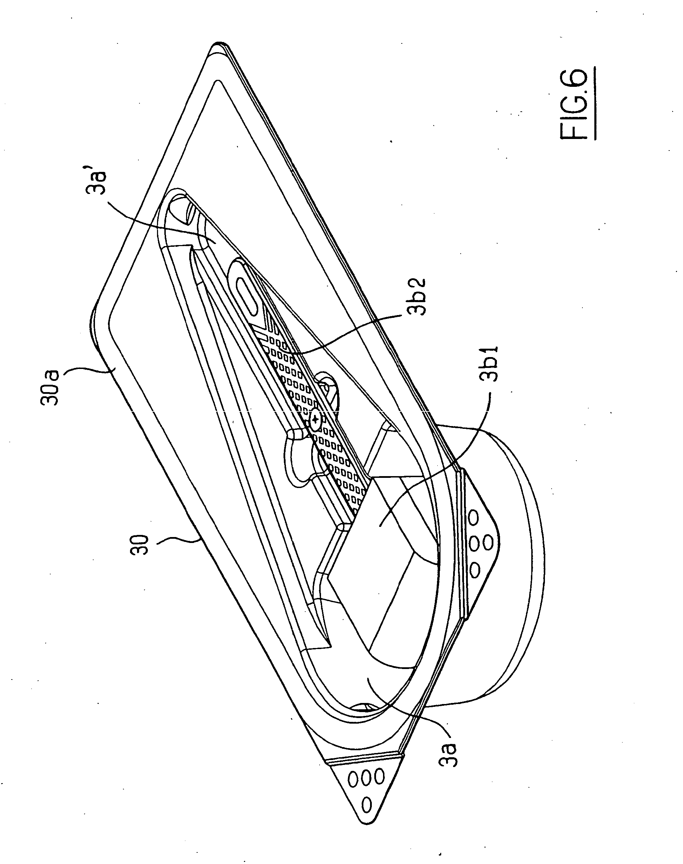

- la figure 6 est un schéma de l'ensemble de matériel de badigeonnage de la figure 5 dans son conteneur individuel fermé par un film transparent.

On a déjà proposé des matériels présentés dans des blisters (coques plastiques plus ou moins rigides) (par exemple US 3 976 195, US 5 779 053, US 6 248 085) en vue de réaliser des applications de liquides en chirurgie.

La présente invention préconise également mais non exclusivement l'utilisation de blisters.

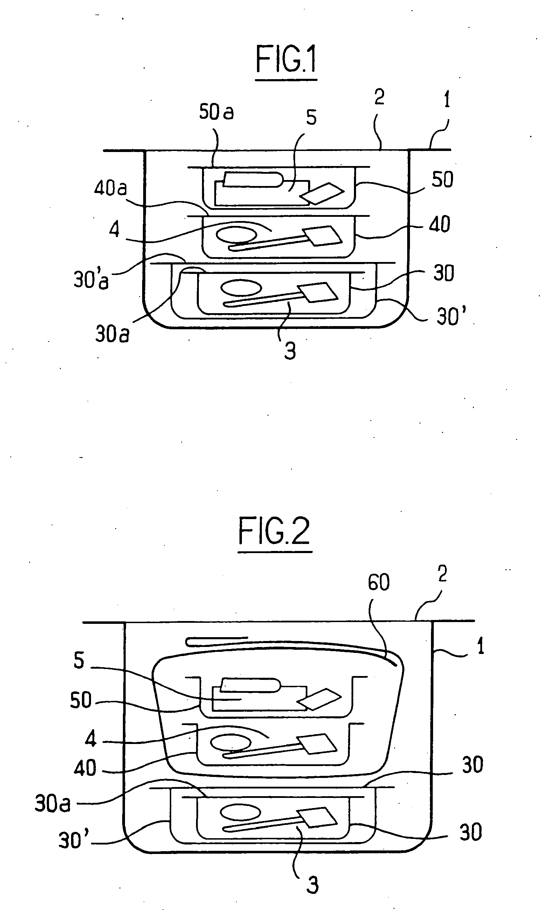

Le dispositif représenté sur la figure 1 comprend, dans un blister stérile (1) fermé par un film pelable (2), un empilage constitué, du bas vers le haut, d'un premier matériel de badigeonnage (3) contenu dans un conteneur (30), d'un deuxième matériel de badigeonnage (4) contenu dans un conteneur (40) et d'un matériel de détersion (5) contenu dans un conteneur (50).

Le premier matériel de badigeonnage est destiné au badigeonnage préliminaire par l'infirmière non stérile et le deuxième matériel de badigeonnage est destiné au badigeonnage final par l'infirmière stérile.

Le matériel de détersion (5) est constitué, dans cet exemple, d'une cupule (5a) pour recevoir un produit de lavage, d'un gant de toilette (5b) pour nettoyer la peau du patient avec ce liquide sur et largement autour du site opératoire, d'une brosse pour nettoyer (5b') pour les parties plus difficiles à nettoyer et des serviettes (5c) pour sécher la peau.

Ce matériel est placé dans une coque plastique transparente semi-rigide (50) (blister) conformée pour présenter une première cavité (5a) apte à constituer la cupule et à recevoir la brosse (5b') pour nettoyer et une deuxième cavité (5a') apte à loger les serviettes (5c) et le gant (5b).

Le blister (50) est fermé par un film pelable transparent (50a).

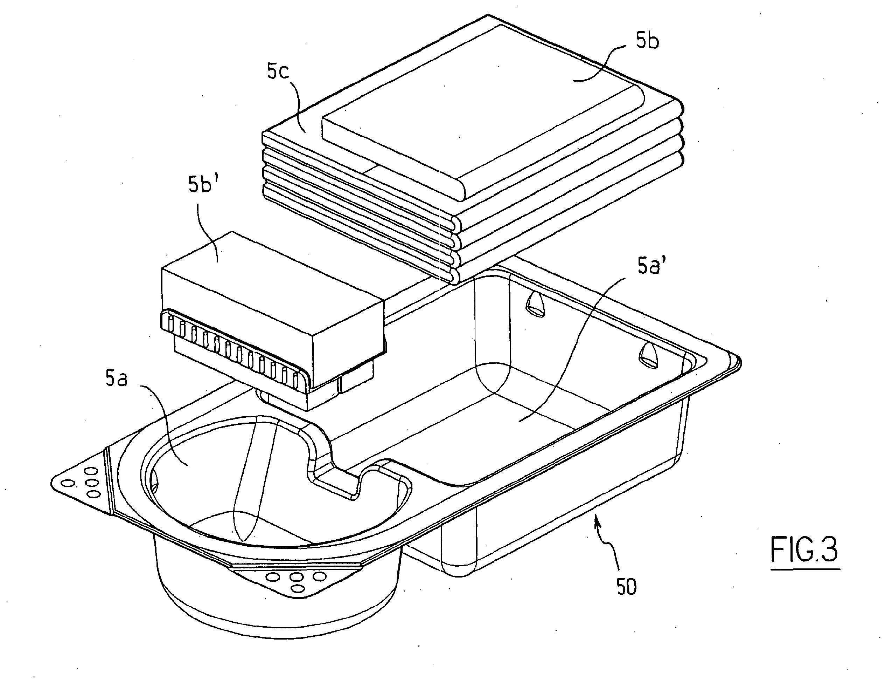

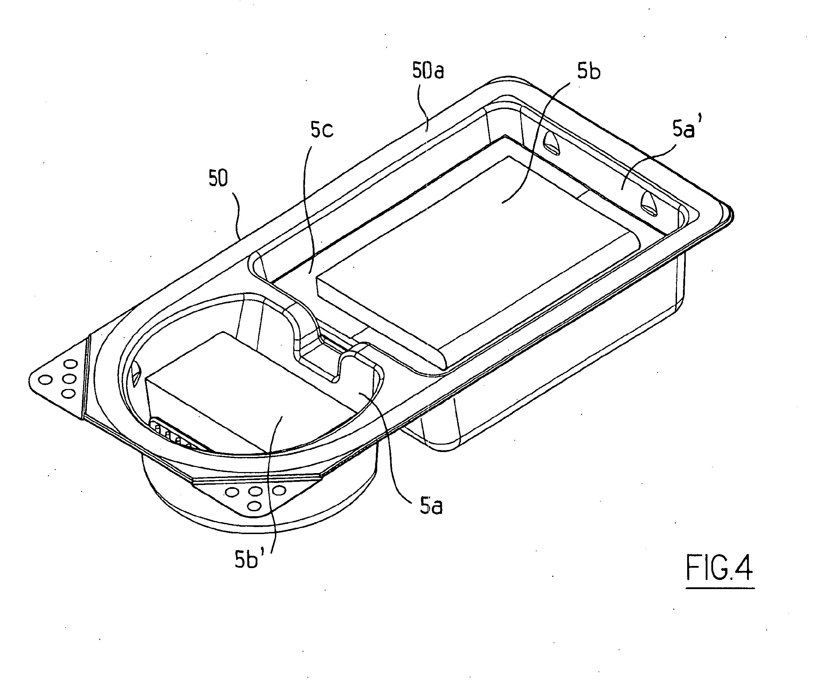

Ces dispositions apparaissent en détails sur les figures 3 et 4.

Le matériel (3) pour l'opération finale de badigeonnage est constitué dans cet exemple d'une cupule (3a) pour recevoir un produit désinfectant, et d'une brosse pour badigeonner (3b) à tremper dans ce produit, cette brosse comprenant un patin (3b1) et un manche (3b2).

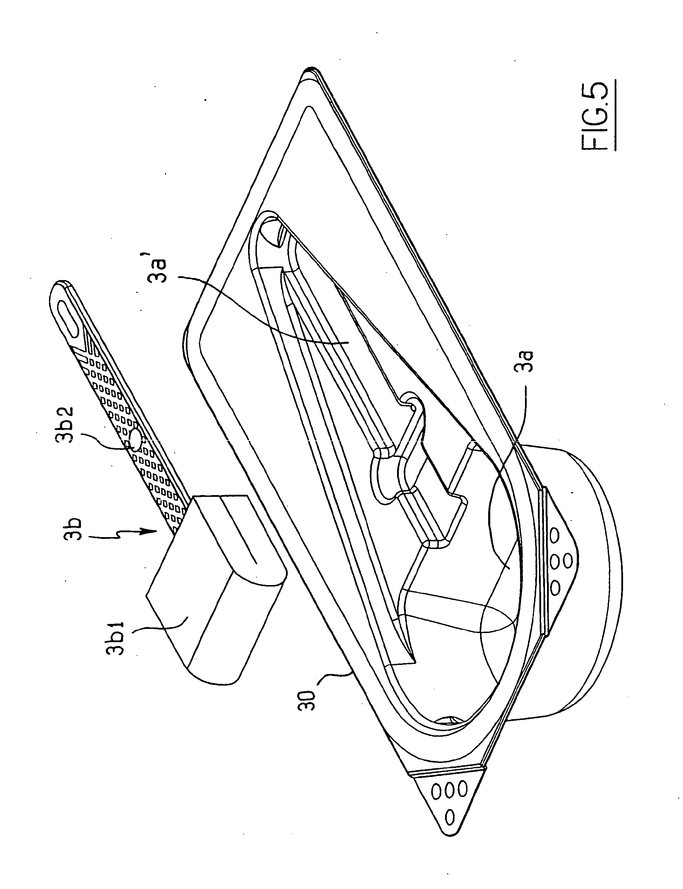

Ce matériel est contenu dans une coque plastique transparente semi-rigide (30) (blister) qui est conformé pour présenter une cavité (3a) apte à constituer la cupule et à loger le patin (3b1) de la brosse pour badigeonner, et une autre cavité (3a') apte à loger le manche (3b2) de la brosse pour badigeonner. Le blister (30) est fermé par un film pelable transparent (30a) (figures 4 et 5).

Le blister (30) est lui-même contenu dans un blister (30') fermé par un film pelable (30'a).

Le matériel (4) pour l'opération préliminaire de badigeonnage est identique au matériel (3) mais il n'est contenu que dans un blister (40) fermé par un film pelable (40a).

Les exemples de matériel qui ont été décrits ne sont pas limitatifs et, dans la pratique, les matériels et leurs conteneurs peuvent varier selon les habitudes, protocoles ou exigences locales.

La figure 2 représente une variante de réalisation du dispositif dans laquelle le conteneur (50) du matériel de détersion et le conteneur (40) du matériel pour l'opération préliminaire de badigeonnage ne sont pas fermés mais sont enveloppés ensemble par un champ opératoire (60) en non-tissé.

L'invention n'est pas limitée à ces exemples de réalisations de l'invention, mais est limitée à l'étendue des revendications.

## Revendications

1. Dispositif pour la préparation d'un site cutané au bloc opératoire qui comprend un matériel de détersion (5) pour le lavage du site, un matériel (4) pour une opération préliminaire de badigeonnage, et un matériel (3) pour une opération finale de badigeonnage, en ce que ces matériels sont placés dans des conteneurs individuels (50 ; 40 ; 30) qui sont réunis dans un conteneur d'ensemble (1) dans des conditions stériles, **charactérisé en ce que** le conteneur (30) du matériel (3) destiné à l'opération finale de badigeonnage est scellé et isolé dans une enveloppe individuelle (30') scellée, en sorte que les opérations préliminaires de détersion et de badigeonnage du site puissent être pratiquées par un intervenant non stérile et en ce que l'opération finale de badigeonnage du site puisse être pratiquée par un intervenant stérile.

2. Dispositif selon la revendication 1 dans lequel le matériel de détersion (5) comprend une cupule (5a) pour recevoir un produit de lavage, un gant de toilette (5b) pour nettoyer la peau du patient avec ce liquide sur et largement autour du site opératoire, une brosse (5b') pour nettoyer pour les parties plus difficiles à nettoyer et des serviettes (5c) pour sécher la peau.

3. Dispositif selon la revendication 2 dans lequel le conteneur du matériel de détersion (5) est un bac (50) conformé pour présenter une première cavité (5a) apte à constituer la cupule et à recevoir la brosse (5b') pour nettoyer et une deuxième cavité (5a') apte à loger les serviettes (5c) et le gant (5b).

4. Dispositif selon la revendication 3 dont le bac (50) est fermé par un film pelable transparent (50a).

5. Dispositif selon la revendication 1 dans lequel chacun des matériels de badigeonnage (3 ; 4) comprend une cupule (3a) pour recevoir un produit désinfectant et une brosse (3b) pour badigeonner à tremper dans ce produit, cette brosse comprenant un patin (3b1) et un manche (3b2).

6. Dispositif selon la revendication 5 dans lequel le conteneur de chaque matériel de badigeonnage est un bac (30 ; 40) conformé pour présenter une cavité (3a) apte à constituer la cupule et à loger le patin (3b1) de la brosse pour badigeonner, et une autre cavité (3a') apte à loger le manche (3b2) de la brosse pour badigeonner.

7. Dispositif selon la revendication 6 dans lequel chaque bac (30 ; 40) contenant un matériel de badigeonnage est fermé par un film pelable ou déchirable transparent (30a ; 40a).

8. Dispositif selon la revendication 6 ou 7 dans lequel l'un des bacs qui contient un matériel de badigeonnage est lui-même contenu dans un bac (30') fermé par un film pelable ou déchirable (30'a).

9. Dispositif selon la revendication 1 dans lequel le matériel de détersion (5) et l'un (4) des matériels de badigeonnage sont contenus dans des bacs individuels (50, 40) qui ne sont pas fermés mais sont enveloppés ensemble par un champ opératoire (60) en non-tissé.

10. Dispositif selon la revendication 1 dans lequel le matériel de détersion (5) comprend une cupule (5a) pour recevoir un produit de lavage, un gant de toilette (5b) pour nettoyer la peau du patient avec ce liquide sur et largement autour du site opératoire, une brosse (5b') pour nettoyer les parties plus difficiles à nettoyer et des serviettes (5c) pour sécher, et dans lequel chacun des matériels de badigeonnage (3 ; 4) comprend une cupule (3a) pour recevoir un produit désinfectant et une brosse (3b) pour badigeonner à tremper dans ce produit, cette brosse pour badigeonner comprenant un patin (3b1) et un manche (3b2).

11. Dispositif selon la revendication 10 dans lequel le conteneur du matériel de détersion (5) est un bac (50) conformé pour présenter une première cavité (5a) apte à constituer la cupule et à recevoir la brosse (5b') pour nettoyer et une deuxième cavité (5a') apte à loger les serviettes (5c) et le gant (5b), et dans lequel le conteneur de chaque matériel de badigeonnage est un bac (30 ; 40) conformé pour présenter une cavité (3a) apte à constituer la cupule et à loger le patin (3b1) de la brosse, pour badigeonner et une autre cavité (3a') apte à loger le manche (3b2) de la brosse pour badigeonner.

12. Dispositif selon la revendication 11 dans lequel le bac (50) contenant le matériel de détersion et les bacs (30,40) contenant chacun un matériel de badigeonnage sont fermés chacun par un film pelable ou déchirable.

13. Dispositif selon la revendication 10 dans lequel l'un des bacs qui contient un matériel de badigeonnage est lui-même contenu dans un bac (30') fermé par un film pelable ou déchirable (30'a).

14. Dispositif selon la revendication 10 ou 11 dans lequel le matériel de détersion (5) et l'un (4) des matériels de badigeonnage sont contenus dans des bacs individuels (50, 40) qui ne sont pas fermés mais sont enveloppés ensemble par un champ opératoire (60) en non-tissé.

15. Dispositif selon l'une des revendications 1 à 14 dans lequel le conteneur d'ensemble (1) est un bac fermé par un film pelable.

16. Dispositif selon la revendication 15 dans lequel sont empilés dans le conteneur d'ensemble (1), du bas vers le haut le conteneur (30) du matériel (3) pour l'opération finale de badigeonnage, le conteneur (40) du matériel (4) pour l'opération préliminaire de badigeonnage et le conteneur (50) du matériel de détersion (5).

17. Dispositif selon l'une des revendications 1 à 16 dans lequel lesdits bacs sont en matériau de synthèse

## Claims

1. A device for preparing a cutaneous site in an operating block, comprising a cleaning material (5) for cleaning the site, a material (4) for a preliminary operation of painting, and a material (3) for a final operation of painting, in that these materials are arranged in individual containers (50 ; 40 ; 30) which are collected together in a general container (1) in sterile conditions, **characterized in that** the container (30) of the material (3) provided for the final operation of painting is sealed and separated in an individual sealed wrapping (30'), in such a way that the preliminary operations of cleaning and painting of the site could be carried out by a non-sterile operator and **in that** the final operation of painting of the site could be carried out by a sterile operator.

2. A device according to claim 1, wherein the cleaning material (5) includes a cup (5a) for receiving the cleaning product, a facecloth (5b) for cleaning the skin of the patient with this liquid over and widely around the operating site, a brush (5b') for cleaning the harder parts to be cleaned and some towels (5c) for drying the skin.

3. A device according to claim 2, wherein the container of the cleaning material (5) is a tray (50) formed to have a first cavity (5a) suitable for constituting the cup and for receiving the brush (5b') provided to clean and a second cavity (5a') suitable for housing the towels (5c) and the facecloth (5b).

4. A device according to claim 3, wherein the tray (50) is closed with a transparent peelable film (50a).

5. A device according to claim 1, wherein each of these painting materials (3 ; 4) comprises a cup (3a) for receiving a sanitizing product and a painting brush (3b) to be dipped in this product, this brush comprising a pad (3b1) and a handle (3b2).

6. A device according to claim 5, wherein the container of each painting material is a tray (30 ; 40) formed to have a cavity (3a) suitable for constituting the cup and for housing the pad (3b1) of the painting brush, and another cavity (3a') suitable for housing the handle (3b2) of the painting brush.

7. A device according to claim 6, wherein each tray (30 ; 40) containing a painting material is closed with a transparent peelable or tearable film (30a ; 40a).

8. A device according to claim 6 or 7, wherein one of the trays which contains a painting material is contained itself in a tray (30') closed with a peelable or tearable film (30'a).

9. A device according to claim 1, wherein the cleaning material (5) and one (4) of the painting materials are contained in individual trays (50, 40) which are not closed but wrapped together with a nonwoven draping (60).

10. A device according to claim 1, wherein the cleaning material (5) comprises a cup (5a) for receiving the cleaning product, a facecloth (5b) for cleaning the skin of the patient with this liquid over and widely around the operating site, a brush (5b') for cleaning the harder parts to be cleaned and some towels (5c) for drying, and wherein each of the painting materials (3 ; 4) comprises a cup (3a) for receiving a sanitizing product and a painting brush (3b) to be dipped in this product, this painting brush comprising a pad (3b1) and a handle (3b2).

11. A device according to claim 10, wherein the container of the cleaning material (5) is a tray (50) formed to have a first cavity (5a) suitable for constituting the cup and for receiving the brush (5b') for cleaning, and a second cavity (5a') suitable for housing the towels (5c) and the facecloth (5b), and wherein the container of each painting materials is a tray (30 ; 40) formed to have a cavity (3a) suitable for constituting the cup and for housing the pad (3b1) of the painting brush, and another cavity (3a') suitable for housing the handle (3b2) of the painting brush.

12. A device according to claim 11, wherein the tray (50) containing the cleaning material and the trays (30, 40) each containing a painting material are each closed with a peelable or tearable film.

13. A device according to claim 10, wherein one of the trays which contains a painting material is contained itself in a tray (30') closed with a peelable or tearable film (30'a).

14. A device according to claim 10 or 11, wherein the cleaning material (5) and one (4) of the painting materials are contained in individual trays (50, 40) which are not closed, but are wrapped together with a nonwoven draping (60).

15. A device according to any one of the claims 1 to 14, wherein the general container (1) is a tray closed with a peelable film.

16. A device according to claim 15, wherein, are stacked, from bottom to top, the container (30) of the material (3) provided for the final operation of painting, the container (40) of the material (4) provided for the preliminary operation of painting, and the container (50) of the cleaning material (5).

17. A device according to any one of the claims 1 to 16, wherein said trays are made in man-made material.

## Patentansprüche

1. Vorrichtung zur Vorbereitung einer Hautstelle im Operationssaal, die ein Detersionsmaterial (5) zum Spülen der Stelle umfasst, ein Material (4) für eine vorläufige Auftragungsoperation und ein Material (3) für eine abschließende Auftragungsoperation, wobei diese Materialien in einzelnen Behältern (50 ; 40 ; 30) platziert sind, die in einem Gesamtbehälter (1) unter sterilen Bedingungen vereint sind, **dadurch gekennzeichnet, dass** der Behälter (30) des Materials (3), das zur abschließenden Auftragungsoperation bestimmt ist, in einer einzelnen versiegelten Hülle (30') versiegelt und isoliert ist, damit die vorläufige Detersions- und Auftragungsoperation der Stelle von einem nicht-sterilen Teilnehmer vorgenommen werden kann und dass die abschließende Auftragungsoperation der Stelle von einem sterilen Teilnehmer vorgenommen werden kann.

2. Vorrichtung gemäß Anspruch 1, bei der das Detersionsmaterial (5) eine Schale (5a) umfasst, um ein Spülprodukt aufzunehmen, einen Waschlappen (5b), um die Haut des Patienten mit dieser Flüssigkeit auf und weitgehend um die Operationsstelle herum zu reinigen, eine Bürste (5b'), um die schwieriger zu reinigenden Partien zu reinigen, und Handtücher (5c), um die Haut zu trocknen.

3. Vorrichtung gemäß Anspruch 2, bei der der Behälter des Detersionsmaterials (5) eine Wanne (50) ist, die ausgelegt ist, um einen ersten Hohlraum (5a) darzustellen, der geeignet ist, die Schale zu bilden und die Reinigungsbürste (5b') aufzunehmen, sowie einen zweiten Hohlraum (5a'), der geeignet ist, um Platz für die Handtücher (5c) und den Handschuh (5b) zu bieten.

4. Vorrichtung gemäß Anspruch 3, deren Wanne (50) von einer abziehbaren transparenten Folie (50a) verschlossen ist.

5. Vorrichtung gemäß Anspruch 1, bei der jedes der Auftragungsmaterialien (3 ; 4) eine Schale (3a) umfasst, um ein desinfizierendes Produkt aufzunehmen, und eine Auftragungsbürste (3b), um in dieses Produkt einzutauchen, wobei diese Bürste einen Kopf (3b1) und einen Griff (3b2) umfasst.

6. Vorrichtung gemäß Anspruch 5, bei der der Behälter jedes Auftragungsmaterials eine Wanne (30 ; 40) ist, die ausgelegt ist, um einen Hohlraum (3a) darzustellen, der geeignet ist, die Schale zu bilden und Platz für den Kopf (3b1) der Auftragungsbürste zu bieten, sowie einen weiteren Hohlraum (3a'), der geeignet ist, Platz für den Griff (3b2) der Auftragungsbürste zu bieten.

7. Vorrichtung gemäß Anspruch 6, bei der jede Wanne (30 ; 40), die ein Auftragungsmaterial enthält, durch eine abziehbare oder aufreißbare transparente Folie (30a; 40a) verschlossen ist.

8. Vorrichtung gemäß Anspruch 6 oder 7, bei der die eine der Wannen, die ein Auftragungsmaterial enthält, selbst in einer Wanne (30') enthalten ist, die von einer - abziehbaren oder zerreißbaren Folie (30'a) verschlossen ist.

9. Vorrichtung gemäß Anspruch 1, bei der das Detersionsmaterial (5) und das eine (4) der Auftragungsmaterialien in einzelnen Wannen (40, 50) enthalten sind, die nicht verschlossen sind, sondern zusammen von einem chirurgischen Tuch (60) aus Vlies umwickelt sind.

10. Vorrichtung gemäß Anspruch 1, bei der das Detersionsmaterial (5) eine Schale (5a) umfasst, um ein Spülprodukt aufzunehmen, einen Waschlappen (5b), um die Haut des Patienten mit dieser Flüssigkeit auf und weitgehend um die Operationsstelle herum zu reinigen, eine Bürste (5b'), um die schwieriger zu reinigenden Partien zu reinigen und Handtücher (5c) zum Trocknen, und bei der jedes der Auftragungsmaterialien (3 ;4) eine Schale (3a) umfasst, um ein desinfizierendes Produkt und eine Auftragungsbürste (3b), um in dieses Produkt einzutauchen, aufzunehmen, wobei diese Auftragungsbürste einen Kopf (3b1) und einen Griff (3b2) umfasst.

11. Vorrichtung gemäß Anspruch 10, bei der der Behälter des Detersionsmaterials (5) eine Wanne (50) ist, die ausgelegt ist, um einen ersten Hohlraum (5a) darzustellen, der geeignet ist, die Schale zu bilden und die Reinigungsbürste (5b') aufzunehmen, sowie einen zweiten Hohlraum (5a'), der geeignet ist, Platz für die Handtücher (5c) und den Lappen (5b) zu bieten, und bei der der Behälter jedes Auftragungsmaterials eine Wanne (30 ; 40) ist, die ausgelegt ist, um einen Hohlraum (3a) darzustellen, der geeignet ist, die Schale zu bilden und Platz für den Kopf (3b1) der Auftragungsbürste zu bieten, sowie einen weiteren Hohlraum (3a'), der geeignet ist, um Platz für den Griff (3b2) der Auftragungsbürste zu bieten.

12. Vorrichtung gemäß Anspruch 11, bei der die Wanne (50), die das Detersionsmaterial enthält, und die Wannen (30,40), von denen jede ein Auftragungsmaterial enthält, jeweils von einer abziehbaren oder zerreißbaren Folie verschlossen sind.

13. Vorrichtung gemäß Anspruch 10, bei der die eine der Wannen, die ein Auftragungsmaterial enthält, selbst in einer Wanne (30') enthalten ist, die von einer abziehbaren oder zerreißbaren Folie (30' a) verschlossen ist.

14. Vorrichtung gemäß Anspruch 10 oder 11, bei der das Detersionsmaterial (5) und das eine (4) der Auftragungsmaterialien in einzelnen Wannen (50, 40) enthalten sind, die nicht verschlossen sind, sondern zusammen von einem chirurgischen Tuch (60) aus Vlies umwickelt sind.

15. Vorrichtung gemäß einem der Ansprüche 1 bis 14, bei der der Gesamtbehälter (1) eine von einer abziehbaren Folie verschlossene Wanne ist.

16. Vorrichtung gemäß Anspruch 15, bei der im Gesamtbehälter (1) von unten nach oben der Behälter (30) des Materials (3) für die abschließende Auftragungsoperation, der Behälter (40) des Materials (4) für die vorläufige Auftragungsoperation und der Behälter (50) des Detersionsmaterials (5) aufeinander geschichtet sind.

17. Vorrichtung gemäß einem der Ansprüche 1 bis 16, bei der die Wannen aus Synthesematerial sind.
